Europäisches Patentamt

European Patent Office  (11) Publication number: **0 049 019**

Office européen des brevets  **B1**

## (12) EUROPEAN PATENT SPECIFICATION

(45) Date of publication of patent specification: **24.04.85**  (51) Int. Cl.⁴: **A 61 F 13/04**

(21) Application number: **81201068.4**

(22) Date of filing: **25.09.81**

(54) Walking sole to be used under an immobilizing bandage of the lower leg whereby all foot joints are rigid.

| | |
|---|---|
| (30) Priority: **29.09.80 BE 885449** | (73) Proprietor: **SPRONKEN ORTHOPEDIE PROTHESECENTRUM PVBA Grotestraat 46 B-3600 Genk (BE)** |
| (43) Date of publication of application: **07.04.82 Bulletin 82/14** | |
| (45) Publication of the grant of the patent: **24.04.85 Bulletin 85/17** | (72) Inventor: **Spronken, Eugéne Nicolaas Marie Grotestraat 46 B-3600 Genk (BE)** |
| (84) Designated Contracting States: **AT CH DE FR GB IT LI LU NL SE** | (74) Representative: **Lips, Hendrik Jan George, Ir. et al HAAGSCH OCTROOIBUREAU Postbus 97702 NL-2509 GC 's-Gravenhage (NL)** |
| (56) References cited: **US-A-3 802 424 US-A-3 916 538** | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

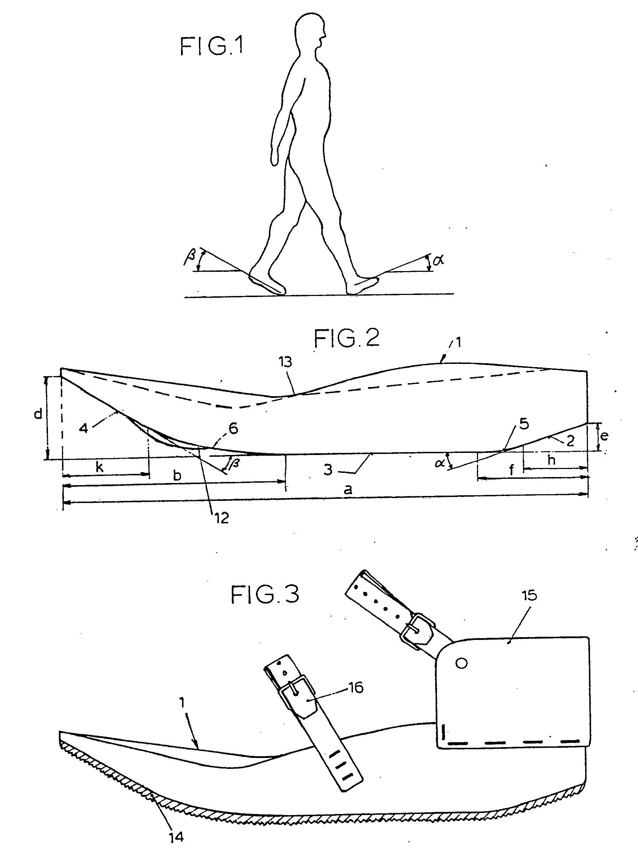

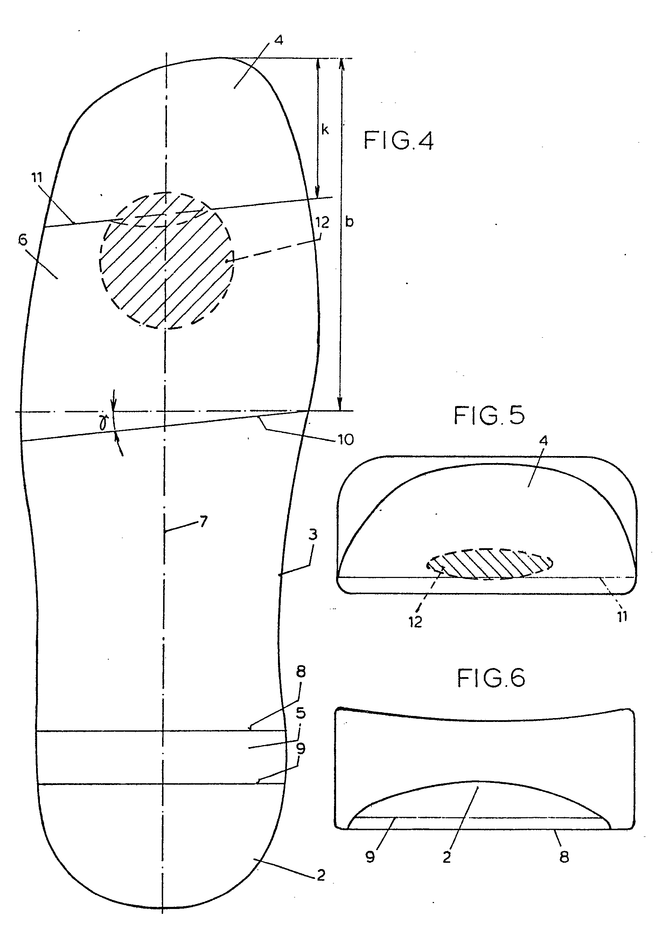

The invention relates to a walking sole to be used under an immobilizing bandage of the lower leg whereby all foot joints are rigid, the running surface of said walking sole consisting of, from front to rear, a foremost portion situated in use under the toes, a centre portion and a hindmost portion, which portions merge into one another with rounded off parts, the foremost and hindmost portions being inclined upwards from the centre portion, and the rear end of the walking sole being approximately at a right angle to the centre portion. Such a walking sole is known from US—A—3802424 and in particular from the Figures 3 and 6. With this known walking sole the upper surface, on which the bandage comes to rest, is essentially flat, whereas the running surface is curved over the entire length, whereby it is aimed to enable a rolling movement of the foot when using the walking sole.

It has appeared, however, that the natural movement of the foot is not circular at all and is also dependent on the length of the foot of the user. Thus a certain instability occurs in the use of the known walking sole.

Further, when the known walking sole is used, the walking sole will be placed on the ground with its rear edge first when the leg concerned is moved forwards, so that a poor first contact with the ground will be established. This not only causes an instability, but also the risk that leg rotations occur.

It is the purpose of the present invention to overcome these drawbacks by providing a walking sole, of which in particular the running surface is shaped in such a way, that the natural movement of the walking process is approached as accurately as possible.

According to the invention this is obtained in that the three portions are essentially flat, that the foremost portion is inclined upwards from the centre portion at an angle between 25 and 35° and the hindmost portion at an angle between 15 and 25°, that the distance between the plane of the centre portion and the highest point of the foremost portion is $(11\pm1)$% and the distance between the plane of the centre portion and the highest point of the hindmost portion is $(3\pm1)$% of the fictive length FL, which equals 1.5×[length a of the sole in mm minus 15 mm], and that the uppermost point of the rear end and that of the front end of the walking sole lie in a plane which is approximately parallel to the centre portion.

In this way a walking sole is obtained, which at the rear end has a much greater thickness than at the front end, as the distance e is considerably shorter than the distance d, whereas the uppermost points at both distances are at approximately the same height above the centre plane.

Therefore the walking sole according to the invention extends from the rear end first almost vertically downwards, to merge then into the inclined portion, which forms an angle of about 20° with the horizontal portion, which inclined portion through a rounded off part merges into the entirely flat centre portion, which again through a rounded off part merges into a foremost portion, which is curved upwards under an angle of 30° with the horizontal plane.

According to a preferable embodiment of the invention, the flat centre portion of the walking sole extends itself between a point at a distance b equal to $(38\pm3)$% FL−$(4\pm1)$ mm from the foremost point of the walking sole and a point at the distance f equal to $(10\pm1)$% FL+$(4\pm1)$ mm from the hindmost point of the walking sole. Here the distance f equals the distance between the hindmost point of the walking sole and the supporting point of the heel. The point at the distance f represents the transition point from the leaning on the heel to the leaning on the entire foot. The movement when the foot is put down, must therefore end in this point. The distance b is determined by the position of the middle foot bone of the big toe.

It has appeared that it is to be preferred that the hindmost inclined flat portion of the walking sole extends until a point at a distance h equal to $(9\pm2)$% FL+$(4\pm1)$ mm from the hindmost point of the walking sole.

Between this point and the earlier-mentioned portion at the distance f, where the flat centre portion begins, a parabolic transition has been provided. Of course such a transition will always be present, although the tolerances of the distances h and f are such that the distances might become equally long.

According to a further elaboration of the invention, the line where the centre portion of the walking sole merges into the rounding off towards the foremost, upwards inclined portion, will begin in a point of the circumferential line of the inside of the sole, that means the side which is turned towards the user's other foot when the walking sole is used, which lies at a distance b from the foremost point of the walking sole and forms an angle with a line at a right angle to the longitudinal direction of the walking sole, of 15° at the most and this in such a way, that the line extends backwards from said point of the inside of the sole.

From the line at the distance b the centre portion merges with a rounding off into the foremost, upwards inclined portion, the rounding off ending in a line at the distance k from the foremost point of the walking sole, this line being parallel to the line passing through the point at the distance b. The distance k is here $(11\pm2)$% of FL.

The invention will now be explained further by means of an example of an embodiment shown in the drawing, in which:

figure 1 schematically shows a side view of a walking person in order to show the angles which play a role in walking;

figure 2 schematically shows a side view of the walking sole according to the invention;

figure 3 shows a side view of the walking sole provided with additional parts such as for fixing the walking sole to the foot;

figures 4, 5 and 6 respectively show a bottom view, a front view and a rear view of the walking sole according to figure 2.

As appears in particular from figure 1, the leg, when moved forwards, forms an angle $\alpha$ with a perpendicular, drawn from the hip joint. If the foot has been fixed under an angle of 90° with respect to the lower leg by means of an immobilizing bandage, the foot will touch the ground under the angle $\alpha$, which angle amounts to 20°±5°.

In connection herewith the hindmost inclined surface 2 of the walking sole 1 shown in figure 2, forms an angle $\alpha$ with the horizontal plane, so that when the forwards extended leg is put down, the surface 2 will have a large contact surface with the ground.

The inclined surface 2 begins at a height e which equals (3±1)% of FL by FL being understood a fictive length, which equal 1.5 (real length a in mm minus 15 mm).

The inclined portion 2 merges with a rounding off into the centre portion 3. This flat centre portion 3 begins at the distance f from the rear end of the walking sole, f equalling (10±1)% of FL+(4±1) mm. The centre portion 3 extends until the distance b from the foremost point of the walking sole, be equalling (38±3)% of FL−(4±1) mm.

Into the foremost point of the centre portion 3 merges the upwards inclined surface 4, which forms an angle $\beta$ with the horizontal plane, said angle $\beta$ equalling 30°±5°.

In view of a good transition the surface 2 merges into the centre portion 3 through the parabolic part 5. The parabolic part 5 of the portion 2 begins at the distance h from the end of the walking sole, h equalling (9±2)% of FL+(4±1) mm.

With the above-mentioned lengths b, f and h the length (4±1) mm corresponds with the thickness of the immobilizing bandage.

As said above, the user of the walking sole, when the leg concerned is put forward, will lean first on the flat portion 2 and then through the transition 5 on the flat portion 3. The user then leans on the whole foot and stands in a stable way, so that he can put the other leg forward without any problem. As soon as this leg leans sufficiently on the ground, the foot provided with the walking sole will swivel with respect to the ground, until the walking sole takes the position in which the foremost surface 4 leans almost entirely on the ground. The highest point of the surface 4 is at a distance d above the horizontal plane, d equalling (11±1)% of FL. At this moment the leg has taken the position as shown in figure 1 for the hindmost leg.

The foremost surface 4 merges through the rounding off 6 into the flat portion 3. The rounding off 6 begins at the distance k from the foremost point of the walking sole, k equalling (11±2)% of FL.

Figure 4 shows a bottom view of the walking sole of Figure 2. The longitudinal direction of the walking sole is indicated by the line 7. The transition lines 8 and 9 between the portions 2 and 3 are at a right angle to the line 7, but the transition lines 10 and 11 between the portions 3 and 4 form an angle $\gamma$ with a line, which is at a right angle to the line 7. The angle $\gamma$ equals 10±5° and is directed in such a way, that the lines 10 and 11 are directed to the outside of the walking sole towards the lines 8 and 9. Thus the foot will make a slightly outwards directed movement, as is also the case in normal walk.

As said above and as appears from figure 4, the line 10 begins in the point of the inside of the walking sole, which lies at the distance b from the foremost point of the walking sole. The line 11 begins in the point of the walking sole at the distance k from the foremost point.

In order to facilitate the turning of the foot, a small protrusion 12, limited by a circle, may be provided in the foremost portion 4 and in particular in the transition part 6 thereof. The protrusion 12 gradually merges at all sides into the adjacent surface.

As appears from figure 2, the foremost point of the upper surface 13 of the walking sole 1 lies approximately at the same height as the hindmost point thereof and the shape of the upper surface 13 has been adapted to the shape of the foot, so that, if the immobilizing bandage is applied over the entire foot in approximately the same thickness, a good support of the bandage and therefore of the foot is obtained.

For an optimal support it will be desirable to manufacture the walking sole in different sizes and also for the left and the right foot. A walking sole of a rigid material will be desired, as otherwise the effect of the specific shape of the lower surface will be lost.

Thus e.g. an embodiment in wood is possible, with which it may be desirable to provide the lower surface with a thin layer of rubber 14 or a similar material, as shown in figure 3, in order to obtain some shock absorption when the foot is put down and also to make the lower surface sufficiently non-slip, so that slipping is avoided.

Figure 3 also shows how the walking sole can be provided with an ankle piece 15 and a foremost strap 16 to connect the walking sole firmly with the foot.

**Claims**

1. Walking sole (1) to be used under an immobilizing bandage of the lower leg whereby all foot joints are rigid, the running surface of said walking sole (1) consisting of, from front to rear a foremost portion (4) situated in use under the toes, a centre portion (3) and a hindmost portion (2), which portions merge into one another with rounded off parts (5, 6), the foremost and hindmost portions (4, 2) being inclined upwards from the centre portion (3), and the rear end of the walking sole (1) being approximately at a right angle to the centre portion (3), characterised in that the three portions (2, 3, 4) are essentially flat, that the foremost portion (4) is inclined upwards from the centre portion (3) at an angle (β) between 25 and 35° and the hindmost portion (2) at an angle (α) between 15 and 25°, that the distance (d) between the plane of the centre portion (3) and the highest point of the foremost portion (4) is (11±1)% and the distance (e) between the plane of the centre portion (3) and the highest point of the hindmost portion (2) is (3±1)% of the fictive length FL, which equals 1.5×[length (a) of the sole in mm minus 15 mm], and that the uppermost point of the rear end and that of the front end of the walking sole (1) lie in a plane which is approximately parallel to the centre portion (3).

2. Walking sole according to claim 1, characterised in that the flat centre portion (3) of the walking sole (1) extends itself between a point at a distance (b) equal to (38±3)% FL−(4±1) mm from the foremost point of the walking sole and a point at the distance (f) equal to (10±1)% FL+(4±1) mm from the hindmost point of the walking sole.

3. Walking sole according to claims 1 or 2, characterised in that the hindmost inclined flat portion (2) of the walking sole (1) extends until a point at a distance (h) equal to (9±2)% FL+(4±1) mm from the hindmost point of the walking sole.

4. Walking sole according to claim 3, characterised in that between the point at the distance (h) and the point at the distance (f) a parabolic transition (5) has been provided between the hindmost flat portion (2) and the centre flat portion (3).

5. Walking sole according to anyone of the claims 2—4, characterised in that the line (10) where the centre portion (3) of the walking sole merged into the rounding off (6) towards the foremost, upwards inclined portion (4), will begin in a point of the circumferential line of the inside of the sole, the side which is turned towards the user's other foot when the walking sole is used, which lies at the distance (b) from the foremost point of the walking sole and forms an angle (γ) with a line at a right angle to the longitudinal direction of the walking sole, of 15° at the most and this in such a way, that the line extends backwards from said point of the inside of the sole.

6. Walking sole according to claim 5, characterised in that the rounding off (6) merges into the foremost upwards inclined portion (4) according to a line (11) which is parallel to the line (10) where the centre portion (3) of the walking sole merges into the rounding off (6) towards the foremost portion (4) and which begins in a point of the circumferential line of the inside of the walking sole, which lies at a distance (k) from the foremost point of the walking sole equalling (11±2)% of FL.

7. Walking sole according to any of the preceding claims, characterised in that a small protrusion (12), limited by a circle, is provided in the foremost upwards inclined portion (4) of the walking sole (1) and in particular in the transition part (6) towards the centre portion.

8. Walking sole according to any of the preceding claims, characterised in that the upper surface (13) thereof is adapted to the shape of the foot.

9. Walking sole according to any of the preceding claims, characterised in that the length thereof is adapted to the length of the user's foot.

10. Walking sole according to any of the preceding claims, characterised in that the walking sole is made of a rigid material such as e.g. wood.

11. Walking sole according to claim 10, characterised in that the lower surface of the walking sole is provided with a thin layer of rubber or a similar material (14).

12 Walking sole according to any of the preceding claims, characterised in that it is provided with an ankle piece (15) and a foremost strap (16) for firmly connecting the walking sole with the foot.

## Revendications

1. Semelle de marche (1) devant être utilisée sous un bandage d'immobilisation de la partie inférieure de la jambe, de façon que toutes les articulations du pied soient rigides, la surface active de ladite semelle de marche (1) comprenant, d'avant en arrière, une portion antérieure (4) située, en cours d'utilisation, au-dessous des orteils, une portion centrale (3) et une portion postérieure (2), portions qui se raccordent mutuellement par des parties arrondies (5, 6), la portion antérieure (4) et la portions postérieure (2) étant inclinées en montant à partir de la portion centrale (3), et l'extrémité postérieure de la semelle de marche (1) faisant sensiblement un angle droit avec la portion centrale (3), caractérisée en ce que les trois portions (2, 3, 4) sont pratiquement plates, en ce que la portion antérieure (4) est inclinée vers le haut à partie de la portion centrale (3) suivant un angle (β) compris entre 25 et 35°, tandis que la portion postérieure (2) l'est d'un angle (α) compris entre 15 et 25°, en ce que la distance (d) entre le plan de la portion centrale (3) et le point le plus haut de la portion antérieure (4) est de (11±1)%, et la distance (e) entre le plan de la portion centrale (3) et le point le plus haut de la portion postérieure (2) de (3±1)% de la longueur fictive FL, qui est égale à 1,5×[longueur (a) de la semelle en mm, moins 15 mm], et en ce que le point le plus haut de l'extrémité postérieure et celui de l'extrémité antérieure de la semelle de marche (1) se trouvent dans un plan sensiblement parallèle à la portion centrale (3).

2. Semelle de marche suivant la revendication 1, caractérisée en ce que la partie centrale plate (3) de la semelle de marche (1) s'étend entre un point à une distance (B) égale à (38±3)% de FL−(4±1) MM du point postérieur de la semelle de marche et un point à une distance F égale à (10±1)% de FL+(4±1) MM du point postérieur de la semelle de marche.

3. Semelle de marche suivant la revendication 1 ou 2, caractérisée en ce que la portion (2) inclinée plate postérieure de la semelle de marche (1) s'étend depuis un point à une distance (h) égale à (9±2)% de FL+(4±1) MM du point postérieur de la semelle de marche.

4. Semelle de marche selon la revendication 1, caractérisée en ce qu'on a ménagé entre la point situé à la distance (h) et le point situé à la distance (f) une transition parabolique (5) entre la portion plate postérieure (2) et la portion plate centrale (3).

5. Semelle de marche selon l'une quelconque des revendications 2 à 4, caractérisée en ce que la ligne (10) où la portion centrale (3) de la semelle de marche se raccorde avec la partie arrondie (6) vers la portion antérieure inclinée vers le haut (4), commence en un point de la ligne circonférentielle de l'intérieur de la semelle, côté qui est tourné vers l'autre pied de l'utilisateur au cours de l'utilisation de la semelle de marche, qui se trouve à la distance (b) du point le plus en avant de la semelle de marche et forme un angle (γ) avec une ligne perpendiculaire à la direction longitudinale de la semelle de marche égale à 15° au plus et cela d'une façon telle que la ligne est dirigée vers l'arrière à partir dudit point de l'intérieur de la semelle.

6. Semelle de marche selon la revendication 5, caractérisée en ce que la partie arrondie (6) se raccorde avec la portion antérieure inclinée vers le haut (4) suivant une ligne (111) qui est parallèle à la ligne (10) au niveau de laquelle la portion centrale (3) de la semelle de marche se raccorde avec la partie arrondie (6) vers la portion antérieure (4) et qui commence en un point du pourtour de l'intérieur de la semelle de marche qui se trouve à une distance (k) du point situé le plus en avant de la semelle de marche égale à (11±2)% de FL.

7. Semelle de marche selon l'une quelconque des revendications précédentes, caractérisée en ce qu'une petite saillie (12), limitée par un cercle, est présente dans la portion antérieure inclinée vers le haut (4) de la semelle de marche (1) et, en particulier, dans la partie de transition (6) vers la portion centrale.

8. Semelle de marche selon l'une quelconque des revendications précédentes, caractérisée en ce que sa surface supérieure (13) est adaptée à la forme du pied.

9. Semelle de marche selon l'une quelconque des revendications précédentes, caractérisée en ce que sa longueur est adaptée à la longueur du pied de l'utilisateur.

10. Semelle de marche selon l'une quelconque des revendications précédentes, caractérisée en ce que la semelle de marche est constituée par une matière rigide, comme par exemple du bois.

11. Semelle de marche selon la revendication 10, caractérisée en ce que la surface inférieure de la semelle de marche comporte une couche mince de caoutchouc ou d'une matière analogue (14).

12. Semelle de marche selon l'une quelconque des revendications précédentes, caractérisée en ce qu'elle comporte une pièce cheville (15) et une sangle antérieure (16) pour maintenir fermement la semelle de marche sur le pied.

**Patentansprüche**

1. Laufsohle (1) zur Benutzung unter einem immobilisierenden Verband des Unterbeins, wobei alle Fusselenke steif sind, von welcher Laufsohle (1) die Laufflüche von vorn nach hinten aus einem vorderen Teil (4), der während der Benutzung unter den Zehen liegt, einem mittleren Teil (3) und einem hinteren Teil (2) besteht, welche Teil mit abgerundeten Teilen (5, 6) ineinander übergehen, wobei der vordere und hintere Teil (4, 2) von dem mittleren Teil (3) ab nach oben neigen und das hintere Ende der Laufsohle (1) nahezu winkelrecht auf dem mittleren Teil (3) steht, dadurch gekennzeichnet, dass die drei Teile (2, 3, 4) im wesentlichen flach sind, der vordere Teil (4) von dem mittleren Teil (3) ab unter einem Winkel (β) zwischen 25 und 35° und der hintere Teil (2) unter einem Winkel (α) zwischen 15 und 25° nach oben neigen, der Abstand (d) zwischen der Fläche des mittleren Teiles (3) und dem höcksten Punkt des vorderen Teiles (4) (11±1)% und der Abstand (e) zwischen der Fläche des mittleren Teiles (3) und dem höcksten Punkt des hinteren Teiles (2) (3±1)% der fiktiven Länge FL ist, die 1,5×[(Länge (a) der Sohle in mm minus 15 mm)] ist und der höchste Punkt des hinteren Endes und des vorderen Endes der Laufsohle (1) in einer Ebene liegen, die etwa parallel zu dem Mittelteil (3) verläuft.

2. Laufsohle gemäss Anspruch 1, dadurch gekennzeichnet, dass das Flache Mittelteil (3) der Laufsohle (1) sich zwischen einem Punkt in einem Abstand b gleich (38±3)% FL−(4±1) mm von dem vorderen Punkt der Laufsohle ab und einem Punkt in einem Abstand f gleich (10±1)% FL+(4±1) mm von dem hinteren Punkt der Laufsohle ab erstreckt.

3. Laufsohle gemäss Anspruch 1 oder 2, dadurch gekennzeichnet, dass der hintere geneigte flache Teil (2) der Laufsohle (1) sich bis zu einem Punkt in einem Abstand (h) gleich (9±2)% FL+(4±1) mm von dem hinteren Punkt der Laufsohle ab erstreckt.

4. Laufsohle gemäss Anspruch 3, dadurch gekennzeichnet, dass zwischen dem Punkt in dem Abstand (h) und dem Punkt in dem Abstand (f) ein prabolisch verlaufender Uebergang (5) zwischen dem hinteren Flachen Teil (2) und dem mittleren flachen Teil (3) vorgesehen ist.

5. Laufsohle gemäss einem der Ansprüche 2—4, dadurch gekennzeichnet, dass die Linie (10) wo der Mittelteil (3) der Laufsohle in die Abdrundung (6) nach dem vorderen, nach oben geneigten Teil (4) hin übergeht, in einem Punkt der Umfangslinie der Innenseite der Sohle anfangen wird, d.h. die Seite die bei der Benutzung der Laufsohle dem anderen Fuss des Benutzers zugekehrt ist, der in dem Abstand (b) von dem vorderen Punkt der Laufsohle liegt und mit einer senkrecht zur Längsrichtung der Laufsohle gerichteten Linie einen Winkel (γ) von höchstens 15° bildet und Zwar derart, dass die Linie sich von dem genannten Punkt der Innenseite der Sohle nach hinten erstreckt.

6. Laufsohle gemäss Anspruch 5, dadurch gekennzeichnet, dass die Abrundung (6) in den nach oben geneigten vorderen Teil (4) gemäss einer Linie (11) übergeht, die parallel zu der Linie (10) verläuft wo der Mittelteil (3) der Laufsohle in die Abrundung (6) nach dem vorderen Teil (4) hin übergeht und die in einem Punkt der Umfangslinie der Innenseite der Laufsohle anfängt der in einem Abstand (k) von dem vorderen Punkt der Laufsohle gleiche (11±2)% von FL liegt.

7. Laufsohle gemäss einem der vorangehenden Ansprüche, dadurch gekennzeichnet, dass ein kleiner von einem Kreis begrenzter Vorsprung (12) in dem nach oben geneigten vorderen Teil (4) der Laufsohle (1) und insbesondere in dem Uebergangsteil (6) nach dem Mittelteil hin vorgesehen ist.

8. Laufsohle gemäss einem der vorangehenden Ansprüche, dadurch gekennzeichnet, dass die obere Fläche (13) derselben an die Form des Fusses angepasst ist.

9. Laufsohle gemäss einem der vorangehenden Ansprüche, dadurch gekennzeichnet, dass die Länge derselben an die Länge des Fusses des Benutzers angepasst ist.

10. Laufsohle gemäss einem der vorangehenden Ansprüche, dadurch gekennzeichnet, dass die Laufsohle aus einem steifen Material, wie z.B. Holz, hergestellt ist.

11. Laufsohle gemäss Anspruch 10, dadurch gekennzeichnet, dass die untere Oberfläche der Laufsohle mit einer dünnen Schicht Gummi oder dergleichen (14) versehen ist.

12. Laufsohle gemäss einem der vorangehenden Ansprüche, dadurch gekennzeichnet, dass sie mit einem Knöchelstück (15) und ein vorderen Riemen (16) zur festen Verbindung der Laufsohle mit dem Fuss versehen ist.

FIG.1

FIG.2

FIG.3

FIG.4

FIG.5

FIG.6